# DEMANDE DE BREVET EUROPEEN

(11) **EP 1 870 086 A1**
(43) Date de publication de la demande: **26.12.2007**
(21) Numéro de dépôt: 07108256.4
(22) Date de dépôt: 15.05.2007
(51) Int. Cl.: A61K 8/42, A61Q 19/00, A61Q 5/00

(54) **Ensemble comprenant une composition acide et une composition à base d'hydroxyalkylurée**

(30) Priorité: 21.06.2006 FR 0652583
(71) Demandeur: L'Oréal, 75008 Paris (FR)
(72) Inventeur: Fonolla Moreno, Angeles, 75013, PARIS (FR); Piot, Bertrand, 75009, PARIS (FR)
(74) Mandataire: Leonard, Armelle

(57) **Abrégé**

La présente invention concerne un ensemble pour le soin de la peau et/ou du cuir chevelu comprenant :
- une composition A contenant un milieu acide physiologiquement acceptable et au moins un agent de soin de la peau et/ou du cuir chevelu ;
- une composition B, conditionnée de manière séparée de la composition A, la composition B contenant un milieu basique physiologiquement acceptable et au moins une hydroxyalkylurée de formule (I)

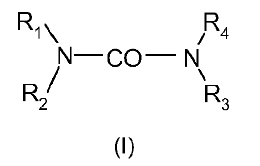

dans laquelle R₁, R₂, R₃ et R₄ représentent chacun indépendamment un atome d'hydrogène, un groupe alkyle en C₁-C₄ ou un groupe hydroxyalkyle en C₂-C₆ pouvant contenir de 1 à 5 groupes hydroxyle, au moins l'un des radicaux R₁-R₄ représentant un groupe hydroxyalkyle, ainsi que leurs sels, solvats et isomères.

## Description

La présente invention concerne un ensemble pour le soin de la peau et/ou du cuir chevelu comprenant :
- une composition A contenant un milieu acide physiologiquement acceptable et au moins un agent de soin de la peau et/ou du cuir chevelu ;
- une composition B, conditionnée de manière séparée de la composition A, la composition B contenant un milieu basique physiologiquement acceptable et au moins une hydroxyalkylurée de formule (I) 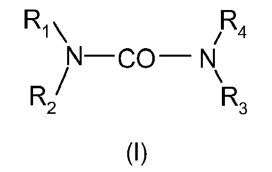
dans laquelle R₁, R₂, R₃ et R₄ représentent chacun indépendamment un atome d'hydrogène, un groupe alkyle en C₁-C₄ ou un groupe hydroxyalkyle en C₂-C₆ pouvant contenir de 1 à 5 groupes hydroxyle, au moins l'un des radicaux R₁-R₄ représentant un groupe hydroxyalkyle, ainsi que leurs sels, solvats et isomères.

On connaît de la demande EP1535607 l'utilisation de l'hydroxyalkylurée de formule (I) 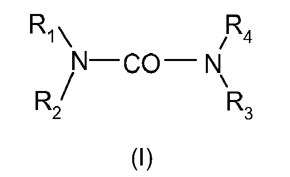 pour ses propriétés hydratantes dans des compositions cosmétiques.
Ces composés donnent également à la composition qui les contient un toucher cosmétique léger, non gras, et d'application facile.
Ces composés sont notamment intéressants dans le soin des peaux sèches et/ou des peaux présentant des zones déshydratées et/ou agressées suite à un traitement, tel qu'un traitement de soin de la peau grasse, un traitement de l'acné, un traitement dépigmentant comprenant notamment l'application sur la peau d'actifs à caractère acide.

Cependant, la Demanderesse a observé que la stabilité de ces composés varie selon le pH. De multiples essais ont en effet montré que ces composés sont stables dans des milieux basiques ayant un pH supérieur ou égal à 7, mais sont instables dans des milieux acides de pH inférieur à 6, en particulier inférieur à 5. Dans ces milieux acides, le pH varie dans le temps et au bout de 15 jours, la Demanderesse a constaté une augmentation de plus d'une unité de pH et une diminution importante de viscosité à 35-45°C.

Ces variations de pH et de viscosité nécessitent donc une formulation des hydroxyalkylurées à un pH basique supérieur ou égal à de 7, ce qui :
- d'une part n'est pas compatible avec certains actifs stables au contraire à pH acide (ex : agents dépigmentants, agents de soin des peaux grasses, agents amincissants...), ou type de formulations ;
- et d'autre part requiert un système de conservation renforcé vis-à-vis des microorganismes, via notamment une augmentation de la teneur en conservateurs, ce qui peut générer, en particulier chez les personnes à peaux sensibles et/ou délicates et/ou agressées, des sensations d'inconfort cutané, telles que des picotements, des rougeurs, des échauffements.

Il subsiste donc le besoin de disposer d'un soin de la peau et/ou du cuir chevelu associant de l'hydroxyalkylurée de formule (I) pour ses propriétés hydratantes et des actifs de soin de la peau et/ou du cuir chevelu à caractère acide, tout en préservant une bonne cosméticité des compositions, c'est-à-dire de bonnes propriétés de stabilité et conservation dans le temps ainsi qu'une bonne efficacité et tolérance sur la peau après application.

La Demanderesse a découvert de façon inattendue qu'un ensemble comprenant :
- une composition A contenant un milieu acide physiologiquement acceptable et au moins un agent de soin de la peau et/ou du cuir chevelu ;
- une composition B, conditionnée de manière séparée de la composition A, la composition B contenant un milieu basique physiologiquement acceptable et au moins une hydroxyalkylurée de formule (1) 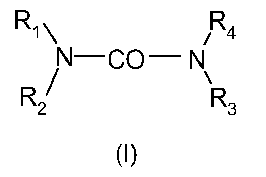
dans laquelle R₁, R₂, R₃ et R₄ représentent chacun indépendamment un atome d'hydrogène, un groupe alkyle en C₁-C₄ ou un groupe hydroxyalkyle en C₂-C₆ pouvant contenir de 1 à 5 groupes hydroxyle, au moins l'un des radicaux R₁-R₄ représentant un groupe hydroxyalkyle, ainsi que leurs sels, solvats et isomères,
répondait à ce besoin.

L'ensemble selon l'invention permet notamment d'appliquer simultanément ou succesivement sur la peau à la fois des agents de soin à caractère acide et une hydroxyalkylurée de formule (I) stable à pH basique, qui ne seraient pas compatibles dans une même composition à pH unitaire.

Par ailleurs, cet ensemble permet de délivrer sur la peau, une composition A et une composition B qui, lors de leur mélange sur la peau, donnent un pH du mélange allant de 4 à 6.5, en particulier un pH proche du pH physiologique (pH de 5.5), qui respecte donc l'état physiologique de la peau.
Cet ensemble est donc adapté pour le soin de tout type de peau et est avantageux pour le soin des peaux sensibles, délicates (ex : peau de bébé) et/ou peaux agressées (ex : rasage, peaux ayant subi un traitement acide de type traitement acnéique, traitement dépigmentant ou traitement de soin de la peau grasse...).

Cet ensemble permet donc d'associer une bonne efficacité des agents de soin présents dans la composition A à une bonne efficacité hydratante de l'hydroxyalkylurée présente dans la composition B.

Comme autre avantage, l'ensemble selon l'invention permet également de diminuer la quantité totale de conservateurs délivrés sur la peau. En effet, le taux de conservateurs pour protéger la composition A à pH acide est miminum, ce qui permet d'avoir, après application sur la peau de la composition A et de la composition B, un mélange présentant un taux de conservateurs moindre par rapport à une formule classique contenant de l'hydroxyalkylurée à pH 7 ou plus. Cette diminution du taux de conservateurs est particulièrement recherchée, notamment pour le soin des peaux sensibles, des peaux délicates et/ou des peaux agressées.

L'invention concerne donc un ensemble pour le soin de la peau et/ou du cuir chevelu comprenant :
- une composition A contenant un milieu acide physiologiquement acceptable et au moins un agent de soin de la peau et/ou du cuir chevelu ;
- une composition B, conditionnée de manière séparée de la composition A, la composition B contenant un milieu basique physiologiquement acceptable et au moins une hydroxyalkylurée de formule (I) 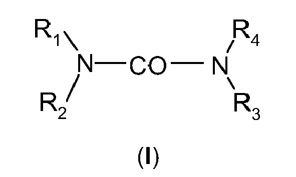
dans laquelle R₁, R₂, R₃ et R₄ représentent chacun indépendamment un atome d'hydrogène, un groupe alkyle en C₁-C₄ ou un groupe hydroxyalkyle en C₂-C₆ pouvant contenir de 1 à 5 groupes hydroxyle, au moins l'un des radicaux R₁-R₄ représentant un groupe hydroxyalkyle, ainsi que leurs sels, solvats et isomères.

Par 'agent de soin de la peau et/ou du cuir chevelu', on entend un agent destiné à embellir l'aspect esthétique de la peau et/ou du cuir chevelu et/ou son confort. En particulier, l'agent peut être destiné à atténuer les irrégularités visibles (dyschromies, hyperpigmentations...) et/ou tactiles (rides, capitons, comédons, cicatrices d'acné...) de la peau et/ou rendre la peau plus saine, moins grasse et/ou moins sèche.

Selon un mode particulier, ledit agent de soin de la peau présent dans la composition A est autre qu'un autobronzant ou une enzyme.

Selon un autre mode particulier de l'invention, ledit agent de soin de la peau est présent dans la composition A en une teneur allant de 0,0001 à 90% en poids par rapport au poids total de la composition.

Par 'milieu physiologiquement acceptable' on entend selon l'invention un milieu compatible avec toutes les matières kératiniques telles que la peau y compris le cuir chevelu, les ongles, les muqueuses, les yeux et les cheveux ou toute autre zone cutanée du corps.

Le milieu selon l'invention est de préférence un milieu aqueux, c'est-à-dire d'un milieu comportant une quantité d'eau d'au moins 5 % en poids, allant de préférence de 30 à 99,4 % en poids et mieux de 30 à 95 % en poids par rapport au poids total de la composition.

Par 'milieu acide' selon l'invention, on entend un milieu notamment aqueux de pH inférieur à 6, en particulier égal ou inférieur à 5.5, et en pratique choisi dans la gamme allant de 1 à 5.5.
En particulier, on utilisera un milieu acide ayant un pH allant de 1 à 5.5, de préférence de 3 à 5,5, et encore plus préférentiellement de 4 à 5.5.

Selon un mode particulier, l'ensemble selon l'invention est caractérisé par un milieu acide ayant un pH inférieur ou égal à 5.

Par 'milieu basique' selon l'invention, on entend un milieu notamment aqueux de pH supérieur ou égal à 7.

### Composition B à pH basique

Les hydroxyalkylurées présentes dans la composition B sont choisies par celles répondant à la formule générale (I) : 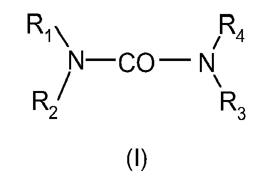 dans laquelle R₁, R₂, R₃ et R₄ représentent chacun indépendamment un atome d'hydrogène, un groupe alkyle en C₁-C₄ ou un groupe hydroxyalkyle en C₂-C₆ pouvant contenir de 1 à 5 groupes hydroxyle, au moins l'un des radicaux R₁-R₄ représentant un groupe hydroxyalkyle, ainsi que leurs sels, solvats et isomères.

Dans la formule (I), parmi les groupes alkyle, on peut notamment citer les groupes méthyle, éthyle, n-propyle, isopropyle, n-butyle, iso-butyle et tert-butyle.

Selon un mode particulier de l'invention, R₁ est un groupe hydroxyalkyle et R₂, R₃ et R₄ représentent, indépendamment les uns des autres, un atome d'hydrogène ou un groupe alkyle en C₁-C₄.

Les composés de formule (I) préférés sont ceux ne renfermant qu'un seul groupe hydroxyalkyle, c'est-à-dire ceux pour lesquels R₁ est un groupe hydroxyalkyle et R₂, R₃ et R₄ représentent, indépendamment les uns des autres, un atome d'hydrogène ou un groupe alkyle en C₁-C₄.

On préfère plus particulièrement les composés de formule (I) pour lesquels R₁ est un groupe hydroxyalkyle et R2, R3 et R4 représentent chacun un atome d'hydrogène.

Parmi les groupes hydroxyalkyle, on préfère ceux contenant un seul groupe hydroxyle et en particulier les groupes hydroxyéthyle, hydroxypropyle, hydroxybutyle, hydroxypentyle et hydroxyhexyle. Le groupe hydroxyéthyle est préféré.

Selon un mode préféré, les composés de formule (I) présents dans la composition B sont choisis parmi la N-(2-hydroxyéthyl)-urée ; la N-(2-hydroxypropyl)-urée ; la N-(3-hydroxypropyl)-urée ; la N-(2,3-dihydroxypropyl)- urée ; la N-(2,3,4,5,6-pentahydroxyhexyl)- urée ; la N-méthyl-N-(1,3,4,5,6-pentahydroxy-2-hexyl)- urée ; la N-méthyl-N'-(1-hydroxy-2-méthyl-2-propyl)- urée ; la N-(1-hydroxy-2-méthyl-2-propyl)- urée ; la N-(1,3-dihydroxy-2-propyl)- urée ; la N-(tris-hydroxyméthyl-méthyl)-urée ; la N-éthyl-N'-(2-hydroxyéthyl)- urée ; la N,N-bis-(2-hydroxyéthyl)- urée ; la N,N'-bis-(2-hydroxyéthyl)-urée ; la N,N-bis-(2-hydroxypropyl)- urée ; la N,N'-Bis-(2-hydroxypropyl)- urée ; la N,N-Bis-(2-hydroxyéthyl)-N'-propyl- urée ; la N,N-Bis-(2-hydroxypropyl)-N'-(2-hydroxyéthyl)-urée ; la N-tert.Butyl-N'-(2-(hydroxyéthyl)-N'-(2-(hydroxypropyl)- urée ; la N-(1,3-dihydroxy-2-propyl)-N'-(2-hydroxyéthyl)- urée ; la N,N-Bis-(2-hydroxyéthyl)-N',N'-dimethyl-urée ; la N,N,N',N'-tetrakis-(2-hydroxyéthyl)- urée ; et la N',N'-Bis-(2-hydroxyéthyl)-N',N'-bis-(2-hydroxypropyl)- urée.

Un composé particulièrement préféré pour une utilisation dans la composition B est la N-(2-hydroxyéthyl)-urée, ci-après désignée par "hydroxyéthyl urée".

Les hydroxyalkylurées de formule (I) peuvent être préparées comme décrit dans la demande DE-27 03 185. Parmi celles-ci, l'hydroxyéthyl urée est en outre disponible dans le commerce, sous forme de mélange à 50% en poids dans l'eau, auprès de la société NATIONAL STARCH sous la dénomination commerciale Hydrovance®.

Parmi les sels, on peut citer les sels d'acides minéraux, tels que l'acide sulfurique, l'acide chlorhydrique, l'acide bromhydrique, l'acide iodhydrique, l'acide phosphorique, l'acide borique. On peut également citer les sels d'acides organiques, qui peuvent comporter un ou plusieurs groupes acide carboxylique, sulfonique, ou phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyle. On peut notamment citer l'acide propionique, l'acide acétique, l'acide téréphtalique, l'acide citrique et l'acide tartrique.

Par solvat, on entend un mélange stoechiométrique dudit composé de formule (I) avec une ou plusieurs molécules d'eau ou de solvant organique, un tel mélange étant issu de la synthèse du composé de formule (I).

L'hydroxyalkylurée ou les hydroxyalkylurées sont de préférence présentes dans la composition B en une teneur efficace pour obtenir l'effet hydratant recherché.
En particulier, on les utilisera en une teneur allant de 0,01 à 50% en poids par rapport au poids total de la composition et plus préférentiellement de 0,1 à 20 % en poids par rapport au poids total de la composition et encore plus préférentiellement de 0,1 à 10% en poids par rapport au poids total de la composition.

### Composition A à pH acide

Le ou les agents de soin de la peau et/ou du cuir chevelu présents dans la composition A sont stables dans un milieu acide tel que défini précédemment, c'est-à-dire que leur efficacité reste pratiquement constante à pH acide.
Mis au contraire dans un milieu à pH basique, ledit agent de soin se destabilise et/ou perd de son efficacité (biologique) dans le temps.

Les agents à caractère acide convenant particulièrement à l'invention sont généralement des acides organiques, mais d'autres agents de soin peuvent également convenir et l'homme du métier saura les choisir en fonction de leur stabilité à pH acide et l'effet recherché sur la peau.

En particulier, l'agent de soin présent dans la composition A pourra notamment être choisi parmi un acide organique, un agent dépigmentant, un agent desquamant, un agent anti-microbien, un agent apaisant et/ou anti-inflammatoire, un agent sébo-régulateur, un agent cicatrisant, un agent astringent, un agent amincissant et leurs mélanges.

Selon un premier mode de réalisation, l'agent de soin sera un acide organique. *
Il sera de préférence choisi dans le groupe constitué par les α-hydroxy-acides, les β-hydroxy-acides, les α-céto-acides, les β-céto-acides, leurs dérivés et leurs mélanges.

En particulier, l'acide organique présent dans la composition A peut être choisi dans le groupe constitué par l'acide ascorbique, l'acide kojique, l'acide caféique, l'acide salicylique et ses dérivés, l'acide citrique, l'acide lactique, l'acide méthyllactique, l'acide glucuronique, l'acide glycolique, l'acide pyruvique, l'acide 2-hydroxy-butanoïque, l'acide 2-hydroxypentanoïque, l'acide 2-hydroxyhexanoïque, l'acide 2-hydroxyheptanoïque, l'acide 2-hydroxyoctanoïque, l'acide 2-hydroxy-nonanoïque, l'acide 2-hydroxydécanoïque, l'acide 2-hydroxyundécanoïque, l'acide 2-hydroxydodécanoïque, l'acide 2-hydroxy-tétradécanoïque, l'acide 2-hydroxy-hexadécanoïque, l'acide 2-hydroxy-octadécanoïque, l'acide 2-hydroxytétra-cosanoïque, l'acide 2-hydroxy-eïcosanoïque, l'acide mandélique, l'acide benzoïque, l'acide phényllactique, l'acide gluconique, l'acide galacturonique, l'acide aleuritique, l'acide ribonique, l'acide tartronique, l'acide tartrique, l'acide malique, l'acide fumarique, l'acide rétinoïque et ses dérivés, l'acide benzène 1,4-di(3-méthylidène 10-camphosulfonique, l'acide urocanique, l'acide 2-phényl benzimidazole 5-sulphonique, l'acide α-(oxo-2-bornylidène-3) toluène-4-sulfonique, l'acide 2-hydroxy-4-méthoxy-5-sulfonique, les extraits végétaux contenant des acides et plus spécialement les extraits de fruits, les dérivés xanthiques acides, l'acide β-glycyrrhétinique, l'acide asiatique et leurs mélanges.

D'autres agents de soin de la peau et/ou du cuir chevelu conviennent également à l'invention. Des exemples d'agents utilisables dans la composition A à pH acide sont listés ci-dessous et classés en fonction du type de soin recherché sur la peau, étant entendu que l'homme du métier pourra les combiner dans un même soin pour obtenir des effets complémentaires visant à embellir l'aspect de la peau et/ou son confort.

L'élément selon l'invention pourra notamment constituer un soin de la peau grasse, un soin de la peau âgée, un soin de la peau sèche, un soin visant à dépigmenter la peau ou atténuer des zones et/ou lésions hyperpigmentées de la peau (ex : cicatrices d'acné), un soin amincissant pour le corps, un soin destiné aux peaux sensibles, aux peaux délicates (ex : bébé), aux peaux agressées (ex : rasage...), un soin de peeling pour le visage ou le corps....

### Soin dépigmentant

Comme agents dépigmentants utilisables dans la composition A selon l'invention, on pourra citer notamment l'acide ascorbique (vitamine C) et ses dérivés et notamment la vit CG, CP et 3-O ethyl vitamine C, l'acide kojique, l'acide tranexamique et ses dérivés, l'acide gentisique et ses dérivés, l'acide dioique, l'acide lipoique, l'acide ellagique, l'acide linoléique et ses dérivés.
De préférence, on utilisera comme agent dépigmentant l'acide ascorbique et ses dérivés, l'acide kojique et ses dérivés.

### Soin de la peau grasse

Comme agents de soin de la peau grasse, on connaît notamment les agents desquamants, les agents antimicrobiens, les agents apaisants, les agents anti-inflammatoires, les agents sébo-régulateurs, les agents antioxydants et leurs mélanges.

De préférence la composition A selon l'invention comprendra comme agent de soin un agent sébo-régulateur, c'est-à-dire un agent capable de réguler l'activité des glandes sébacées.
Selon un mode particulier, la composition A pourra comprendre en outre éventuellement un agent antimicrobien.
De façon encore préférée elle pourra comprendre en plus un agent desquamant.
Et pour augmenter encore l'efficacité et/ou la tolérance de ladite composition A, on pourra rajouter en outre des agents apaisants ou anti-inflammatoires, des agents antioxydants, des agents cicatrisants, des agents astringents, et leurs mélanges.

Comme agents séborégulateurs utilisables dans la composition A selon l'invention, on peut citer notamment :
- l'acide rétinoïque et ses dérivés ;
- les sels de zinc tels que le gluconate de zinc, le pyrrolidone carboxylate de zinc (ou pidolate de zinc), le lactate de zinc, l'aspartate de zinc, le carboxylate de zinc, le salicylate de zinc, le cystéate de zinc ;
- les sels de cuivre, en particulier le pidolate de cuivre ;
- l'acide sébacique, notamment vendu sous la forme d'un gel de polyacrylate de sodium sous la dénomination Sebosoft par la société Sederma ;
- la glycine greffée sur chaîne undécylénique , telle que celle vendue sous la dénomination Lipacide UG OR par la société Seppic ;
- le mélange d'acide oléanolique et d'acide nordihydroguaïarétique , tel que celui vendus sous la forme d'un gel sous la dénomination AC.Net par la société Sederma ;
- l' acide phthalimidoperoxyhexanoïque ;
- l'acide 10-hydroxydécanoïque, et notamment les mélanges d'acide 10-hydroxydécanoïque, d'acide sébacique et de 1,10-décandiol tels que celui vendu sous la dénomination Acnacidol^{®} BG par la société Vincience ;
- les sels d'aluminium, parmi lesquels on peut citer le chlorhydrate d'aluminium, l'aluminium chlorohydrex, l'aluminium chlorohydrex PEG, l'aluminium chlorohydrex PG, l'aluminium dichlorohydrate, l'aluminium dichlorohydrex PEG, l'aluminium dichlorohydrex PG, l'aluminium sesquichlorohydrate, l'aluminium sesquichlorohydrex PEG, l'aluminium sesquichlorohydrex PG, les sels d'alun, l'aluminium sulfate, l'aluminium zirconium octachlorohydrate, l'aluminium zirconium pentachlorohydrate, l'aluminium zirconium tetrachlorohydrate, l'aluminium zirconium trichlorohydrate et plus particulièrement l'aluminium chlorhydrate commercialisé par la société REHEIS sous la dénomination MICRODRY ALUMINUM CHLOROHYDRATE ou par la société GUILINI CHEMIE sous la dénomination ALOXICOLL PF 40. Des sels d'aluminium et de zirconium sont par exemple celui commercialisé par la société REHEIS sous la dénomination REACH AZP-908-SUF , des sel d'aluminium « activés » par exemple celui commercialisé par la société REHEIS sous la dénomination REACH 103 ou par la société WESTWOOD sous la dénomination WESTCHLOR 200.

Par 'agents anti-microbiens', on entend selon l'invention des agents ayant des effets sur la flore spécifique des peaux grasses, tels que par exemple le *P acnes.* Ces effets peuvent être soit bactéricides, soit anti-adhésion bactérienne (prévient et/ou réduit l'adhésion des micro-organismes) soit agissant sur le biofilm des bactéries pour éviter leur multiplication.

Comme agents antimicrobiens utilisables dans la composition A selon l'invention, on peut citer notamment l'acide citrollique, l'acide spérillique, l'acide 10-hydroxy-2 decanoique comme l'Acnacidol P de Vincience, l'acide azélaique, l'arlatone dioic de Unichema, l'acide phthalimidoperoxyhexanoique ou Eureco HC de Chemron Corporation ; l'acide ellagique ; l'acide undécylenique et ses sels, l'acide 3-hydroxy benzoïque, l'acide 4-hydroxy benzoïque, l'acide phytique, l'acide lipoïque, l'acide azélaïque et ses sels, l'acide arachidonique, l'octanoylglycine (Lipacid C8G^{®} de Seppic), l'acide salicylique et ses dérivés.

Par "agent desquamant", on entend tout composé capable d'agir soit directement sur la desquamation en favorisant l'exfoliation soit sur les enzymes impliquées dans la desquamation ou la dégradation des cornéodesmosomes, les glycosidases, la stratum corneum chymotryptic enzym (SCCE) voire d'autres protéases (trypsine, chymotrypsine-like).

Comme agents desquamants utilisables dans la composition A de l'invention, on peut citer notamment :
- les β-hydroxyacides, en particulier l'acide salicylique et ses dérivés (dont l'acide n-octanoyl 5-salicylique) ; les α-hydroxyacides, tels que les acides glycolique, citrique, lactique, tartrique, malique ou mandélique ; l'acide gentisique et ses dérivés ; l'acide cinnamique ; l'acide jasmonique et dérivés comme dans les demandes de brevet EP 1 333 022 et EP 1 333 021 ;
- l'acide (N-2 hydroxyéthylpiperazine-N-2-éthane) sulfonique (HEPES) ; les dérivés de l'acide 2-oxothiazolidine-4-carboxylique (procystéine) ; les dérivés d'acides alpha aminés de type glycine (tels que décrits dans EP-0 852 949, ainsi que le méthyl glycine diacétate de sodium commercialisé par BASF sous la dénomination commerciale TRILON M) ; le (3-hydroxy-2pentylcyclopentyl)acetic acid.

Comme agents desquamants préférés, on pourra citer les beta-hydroxyacides, tel que l'acide n-octanoyl 5-salicylique ; les acides glycolique, citrique, lactique, tartrique, malique ou mandélique ; l'acide (N-2 hydroxyéthylpiperazine-N-2-éthane) sulfonique (HEPES) ; et leurs mélanges.

Comme agents apaisants particuliers utilisables dans la composition A selon l'invention, on peut citer : la caféine et ses dérivés, l'acide b-glycyrrhétinique et ses sels ou dérivés (le stearyl glycyrrhetate, l'acide 3-stéaroyloxy glycyrrhétique, l'acide glycyrrhétinique monoglucuronide) ainsi que les plantes en contenant (ex : Glycyrrhiza glabra), l'acide oléanolique et ses sels, l'acide ursolique et ses sels, l'acide boswellique et ses sels, l'acide bétulinique et ses sels ; un extrait de menthe, en particulier de feuilles de menthe comme le Calmiskin^{®} de Silab, un extrait de rose comme l'Herbasol rose extract, le Stimu-tex AS de Pentapharm.
De préférence, on utilisera un extrait de menthe, en particulier de feuilles de menthe comme le Calmiskin^{®} de Silab, un extrait de rose comme l'Herbasol rose extract, le Stimu-tex AS de Pentapharm, ou l'acide b-glycyrrhétinique et ses sels ou dérivés.

Comme agents anti-inflammatoires particuliers utilisables dans la composition A selon l'invention, on peut citer notamment l'acide azéalique.

Comme agents cicatrisants utilisables dans la composition A selon l'invention, on peut citer notamment certains acides aminés comme l'hydroxyproline, l'arginine, la sérine ; l'allantoïne ; l'acide ximeninique et ses sels tel que acido ximeninico de Indena, l'herbasol citron de Cosmetochem.
Les cicatrisants préférés sont l'hydroxyproline, l'arginine, la sérine et l'allantoïne.

Comme agents astringents (agents permettant de lutter contre la dilatation des follicules sébacés) utilisables dans la composition A selon l'invention, on peut citer notamment les sels d'aluminium, les sels d'alun, l'extrait d'Hammamelis, et leurs mélanges.
Les aluns utilisables sont choisis parmi notamment :
- l'alun de potassium de structure : KAl(SO₄)₂, 12H₂O
- l'alun d'ammonium de structure : NH₄Al(SO₄)₂, 12H₂O
- l'alun de sodium de structure : NaAl(SO₄)₂, 12H₂O
- les sels de sulfate d'aluminium de structure : Al₂(SO₄)₃, n H₂O où n vaut 0 à 27

Les aluns peuvent être sous forme de poudre ou de cristaux ou sous forme solubilisée.
On utilisera plus particulièrement l'alun de potassium, de préférence en une teneur allant de 0,1 à 3% en poids par rapport au poids total de la composition A, de préférence en une teneur allant de 1 à 2% en poids par rapport au poids total de la composition.

Les astringents préférés sont les aluns et l'extrait d'hammamelis.

### Soin amincissant

Comme agents amincissants utilisables dans la composition A selon l'invention, on peut citer notamment la caféine, l'acide nicotinique et ses dérivés tels que plus particulièrement le nicotinate d'alpha-tocophérol et le nicotinate d'hexyle (voir notamment à ce sujet le document EP-A-371 844).

Comme agent de soin préféré présent dans la composition A, on peut citer notamment l'acide ascorbique et ses dérivés, l'acide kojique, les sels de zinc, les sels de cuivre, l'acide n-octanoyl 5 salicylique, l'acide (N-2 hydroxyéthylpiperazine-N-2-éthane) sulfonique, les acides glycolique, citrique, lactique, tartrique, malique ou mandélique, les sels d'alun, un extrait d'Hammamelis, un extrait de rose, un extrait de menthe, l'acide β-glycyrrhétinique, l'allantoine, la sérine, l'arginine, l'hydroxyproline, la caféine, l'acide nicotinique et ses dérivés.

L'agent de soin de la peau et/ou du cuir chevelu sera généralement présent dans la composition A selon l'invention en une teneur efficace pour l'effet recherché, en particulier l'effet dépigmentant, l'effet anti-peau grasse et/ou l'effet amincissant recherché.
A titre d'exemple, l'agent de soin de la peau et/ou du cuir chevelu sera présent dans la composition A en une teneur allant de 0,001 à 90% en poids par rapport au poids total de la composition A, en particulier de 0,01 à 30% en poids par rapport au poids total de la composition A et encore plus préférentiellement de 0,05 à 10%, voire de 0,1 à 1% en poids par rapport au poids total de la composition A.

La composition A pourra comprendre en outre des adjuvants cosmétiques classiques tels que ceux décrits dans la partie galénique quiva suivre. En particulier, elle pourra comprendre des gélifiants stables à pH acide et des agents de ciblage folliculaire adaptés à une formulation en milieu acide.
Ces agents de ciblage folliculaire sont particulièrement avantageux dans le soin des peaux grasses.

Comme gélifiants hydrophiles ou lipophiles, on peut citer notamment les carbopol, les luvigel, l'Hostacerin AMPS, le Simulgel, les Sepigel, les gommes de xanthane, de guar, de cellulose, les alginates et leurs mélanges. On peut citer aussi les hectorites.

Comme agents de ciblage folliculaire, on peut citer des polymères constitutifs particules poreuses. Ces particules ont de préférence un diamètre moyen en volume inférieur ou égal à 10 µm. En effet, de telles particules peuvent pénétrer dans le follicule sébacé par application d'une force mécanique. Cette force mécanique provient généralement d'un massage qui, outre la poussée qu'elle exerce, génère un effet de pompe au niveau du follicule. Les particules atteignent ainsi progressivement le canal folliculaire dans lequel elles sont susceptibles d'absorber le sébum et, le cas échéant, de libérer le composé actif qu'elles portent. Le matériau constitutif des particules est ensuite rejeté grâce à l'écoulement de sébum et/ou à la pousse du poil permettant ainsi d'éviter toute réaction indésirable éventuelle de l'organisme vis-à-vis de ce matériau.
En particulier, on utilisera des particules, notamment sphériques, poreuses, de dimension moyenne en nombre pouvant aller de 0,1 à 50 µm, notamment de 0,1 à 20 µm et tout particulièrement de 0,5 à 10 µm.
Comme particules poreuses préférées, on peut utiliser des particules de polyamide, en particulier de Nylon 6, Nylon 6-6, Nylon 12 ou Nylon 6-12 telles que celles commercialisées par la société ATOFINA sous le nom générique d'« Orgasol ».
Ce système d'encapsulation qui permet un ciblage folliculaire est particulièrement avantageux dans des compositions destinées au traitement des peaux grasses.

Selon un mode particulier de l'invention, pour renforcer et/ou complémenter l'effet de l'agent de soin présent dans la composition A à pH acide selon l'invention, la composition B à pH basique selon l'invention peut également comprendre au moins un agent additionnel de soin connu lui pour être stable à pH basique.

Par 'agent stable à pH basique' selon l'invention, on entend un agent ayant à pH 7 une efficacité (biologique) presque constante dans le temps. Mis au contraire dans un milieu très acide, cet actif perdrait de son efficacité.

L'homme du métier saura choisir parmi des listes d'agents de soin additionnels cosmétiques et/ou dermatologiques ceux qui conviendraient à la composition B à pH basique selon l'invention.

Ces agents additionnels pourront notamment être choisis parmi les agents dépigmentants, les agents antimicrobiens, les agents antitranspirants, les chélateurs de métaux, les protéines hydrolysées, les antioxydants, les vitamines, les agents anti-inflammatoires, les agents anti-irritants ou apaisants, les agents hydratants, les extraits végétaux, les agents améliorant la fonction barrière, les agents matifiants, les charges abrasives ou agents exfoliants, les agents desquamants, les agents sébo-régulateurs, les agents cicatrisants, les agents astringents, les charges, les azurants optiques, les agents de fluorescence, les agents anti-âge, les agents amincissants et leurs mélanges.

Comme agents humectants ou hydratants, on peut citer notamment le glycérol et ses dérivés, le xylitol, le chitosane et ses dérivés, le collagène, le plancton, un extrait d'imperata cylindra commercialisé sous la dénomination Moist 24^{®} par la société Sederma.

Comme agents améliorant la fonction barrière, on peut citer notamment les céramides et dérivés, les composés à base de sphingoïdes, les glycosphingolipides, les phospholipides, le cholestérol et ses dérivés, les phytostérols, les acides gras essentiels, le diacylglycérol, la 4-chromanone et dérivés de chromon, la vaseline, la lanoline, les beures de karité, le cocoa butter, et la lanoline.

Comme agents dépigmentants, on pourra citer notamment l'alpha et la béta arbutine, le lucinol et ses dérivés, le résorcinol et ses dérivés, le D calcium panthéteine sulfonate, la vitamine B3, les céramides et leurs homologues, les dérivés de plantes comme la camomille, la busserole, la famille des aloe (vera, ferox, bardensis), de murier, de scutellaire, sans que cette liste soit exhaustive.

Comme agents matifiants, on peut citer notamment un amidon de riz ou un amidon de maïs, la kaolinite, les silices, le talc, un extrait de graines de potiron, des microbilles de cellulose, des fibres végétales, des fibres synthétiques, en particulier de polyamides, des microsphères de copolymères acryliques expansées, des poudres de polyamides, les poudres de silice, les poudres de polytétrafluoroéthylène, les poudres de résine de silicone, les poudres de copolymères acryliques, les poudres de cire, les poudres de polyéthylène, les poudres d'organopolysiloxane réticulé élastomère enrobées de résine de silicone, les poudres composites de talc/dioxyde de titane/alumine/silice, les poudres de silicates mixtes amorphes, les poudres de polymères acryliques, les particules de silicate et notamment de silicate mixte, et leurs mélanges.

Comme agents sébo-régulateurs ou anti-séborrhéiques, on peut citer :
- les mélanges d'extrait de canelle, de thé et d'octanoylglycine tel que le Sepicontrol A5 TEA de chez Seppic ;
- le mélange de capryloyl glycine, de sarcosine et d'extrait de cinnamomum zeylanicum commercialisé notamment par la société SEPPIC sous la dénomination commerciale Sepicontrol A5^{®};
- des extraits de végétaux des espèces Arnica montana, Cinchona succirubra, Eugenia caryophyllata, Humulus lupulus, Hypericum perforatum, Mentha piperita, Rosmarinus officinalis, Salvia oficinalis et Thymus vulgaris, tous commercialisés par exemple par la société MARUZEN ;
- les extraits de reine des prés (spiraea ulamaria) tel que celui vendu sous la dénomination Sébonormine^{®} par la société Silab ;
- les extraits d'algue laminaria saccharina tel que celui vendu sous la dénomination Phlorogine^{®} par la société Biotechmarine ;
- les extraits de sucre de canne, tel que celui commercialisé sous la dénomination Policasonol^{®} par la société Sabinsa ;
- les mélanges d'extraits de racines de pimprenelle (sanguisorba officinalis/poterium officinale), de rhizomes de gingembre (zingiber officinalis) et d'écorce de cannelier (cinnamomum cassia) tel que celui vendu sous la dénomination Sebustop^{®} par la société Solabia ;
- les extraits de graines de lin tel que celui vendu sous la dénomination Linumine^{®} par la société Lucas Meyer ;
- les extraits de Phellodendron tels que ceux vendu sous la dénomination Phellodendron extract BG par la société Maruzen ou Oubaku liquid B par la société Ichimaru Pharcos ;
- les mélanges d'huile d'argan, d'extrait de serenoa serrulata (saw palmetto) et d'extrait de graines de sésame tel que celui vendu sous la dénomination Regu SEB^{®} par la société Pentapharm ;
- les mélanges d'extraits d'epilobe, de terminalia chebula, de capucine et de zinc biodisponible (microalgues) tel que celui vendu sous la denomination Seborilys^{®} par la société green tech ;
- les extraits de Pygeum afrianum tel que celui vendu sous la dénomination Pygeum afrianum sterolic lipid extract par la société Euromed ;
- les extraits de serenoa serrulata tels que ceux vendus sous les dénomination Viapure Sabal par la société Actives International, ou ceux vendus par la société Euromed ;
- les mélanges d'extraits de plantain, de berberis aquifolium et de salicylate de sodium tels que celui vendu sous la dénomination Seboclear^{®} par la société Rahn ;
- l'extrait de clou de girofle tel que celui vendu sous la dénomination Clove extract Powder par la société Maruzen ;
- l'huile d'argan telle que celle vendue sous la dénomination Lipofructyl^{®} par les Laboratoires Sérobiologiques ;
- les filtrats de protéine lactique tels que celui vendu sous la dénomination Normaseb^{®} par la société Sederma ;
- les extraits d'algue laminaria, tel que celui vendu sous la dénomination Laminarghane^{®} par la société Biotechmarine ;
- les oligosaccharides d'algue laminaria digitata tel que celui vendu sous la dénomination Phycosaccharide AC par la société Codif ;
- l'huile de schiste sulfonée, telle que celle vendue sous la dénomination Ichtyol Pale par la société Ichthyol ;
- les extraits d'ulmaire (spiraea ulmaria) tels que celui vendu sous la dénomination Cytobiol Ulmaire par la société Libiol ;
- les glucomannanes extraits de tubercule de konjac et modifié par des chaînes alkylsulfonates tel que celui vendu sous la dénomination Biopol Beta par la société Arch Chemical ;
- les extraits de Sophora angustifolia, tels que ceux vendus sous la dénomination Sophora powder ou Sophora extract par la société Bioland ;
- les extraits de cinchona succirubra bark tel que celui vendu sous la dénomination le Red bark HS par la société Alban Muller ;
- les extraits de quillaja saponaria tel que celui vendu sous la dénomination Panama wood HS par la société Alban Muller ;
- le citrate de trialkyle(C₁₂-C₁₃) vendu sous la dénomination COSMACOL^{®} ECI par la société Sasol ; le citrate de trialkyle(C₁₄-C₁₅) vendu sous la dénomination COSMACOL^{®} ECL par la société Sasol ;
- les activateurs PPAR-y spécifiques comme ceux décrits dans la demande WO 2005/053632 ;
- des extraits de plantes du genre Silybum ;
- des sapogénines ou extraits végétaux en contenant, en particulier les extraits de Dioscorées riches en diosgénine ;
- la criste marine ;
- des extraits d'Eugenia caryophyllata contenant de l'eugénol et du glucoside d'eugényle, et leurs mélanges.

Comme agents desquamants, on peut citer notamment les oligofucoses l'extrait de Saphora japonica ; le resvératrol, le miel ; les dérivés de sucre tels que l'O-octanoyl-6-D-maltose et la N-acétyl glucosamine.

Comme agents apaisants ou anti-irritants, on peut citer notamment les vitamines E, B5, B3, le sulfate de dextran, un extrait de Paeonia suffruticosa et/ou lactiflora, les phycosshacharides de la société Codif, un extrait de Laminaria saccharina, les extraits de Centella asiatica, l'huile de Canola, les extraits de camomille, l'allantoïne, les huiles insaturées en oméga 3 telles que les huiles de rosier musca, de cassis, d'Ecchium, de poisson, l'huile de calophilum, des extraits de plancton, la capryloyl glycine, le Seppicalm VG^{®} (nymphea alba et sodium palmitoylproline) de Seppic, un extrait de Pygeum, un extrait de Boswellia serrata, un extrait de Centipeda cunnighami, un extrait d'Helianthus annus en particulier l'Hélioxine de Silab, un extrait de Linum usitatissimum comme la Sensiline de Silab, les tocotriénols, les extraits de Cola nitida, le piperonal, un extrait de clou de girofle, un extrait d'Epilobium angustifolium, l'aloe vera, un extrait de Bacopa moniera, les phytostérols, l'eau de bleuet, l'eau de rose, le dextran comme dans Modulène^{®} de Vincience, un extrait de menthe, en particulier de feuilles de menthe comme le Calmiskin^{®} de Silab, les dérivés d'anis, les bactéries filamenteuse comme Vitreoscilla filiformis tel que décrit dans le brevet EP 761 204, un extrait de rose comme l'Herbasol rose extract, le Stimu-tex AS de Pentapharm, les sels alcalino-terreux notamment le strontium, la niacinamide, et leurs mélanges.

Comme agents antioxydants, on peut citer notamment le tocophérol et ses esters, en particulier l'acétate de tocophérol ; le BHT et le BHA.
On peut également citer les polyphénols, l'époigallocathéchines et les extraits naturels en contenant, les anthocyanes, les extraits de romarin, les extraits de feuilles d'olivier, le thé vert, le resvératrol et ses dérivés, le Pycnogénol, l'ergothinéine, la N acétylcystéine, la biotine, les chélatants, l'idébénone, des extraits végétaux come le Pronalen Bioprotect TM de la société Provital, les antiradicalaires comme la vitamine E, le co enzyme Q10, les bioflavonoides, les SOD, le phytantriol, les lignanes, la mélatonine, les pidolates, le gluthation.

Comme agents astringents, on peut citer les extrais de scutellaria, les extraits d'ulmaire, les extraits de reine des près, les extraits de gentiane, les extraits de bardane et leurs mélanges.

Comme agents anti-âge, on peut citer notamment :
- parmi les actifs stimulant les macromolécules épidermiques, telles que la fillagrine et les kératines, on peut citer notamment l'extrait de lupin commercialisé par la société SILAB sous la dénomination commerciale Structurine^{®} ; l'extrait de bourgeons de hêtre Fagus sylvatica commercialisé par la société GATTEFOSSE sous la dénomination commerciale Gatuline^{®} RC; et l'extrait de zooplancton Salina commercialisé par la société SEPORGA sous la dénomination commerciale GP4G^{®}; Tripeptide de Cuivre de PROCYTE ; ; un extrait peptidique de *Voandzeia substerranea* tel que celui commercialisé par la société Laboratoires Sérobiologiques sous la dénomination commerciale Filladyn LS 9397^{®}.
- parmi les agents dermo-décontractants, on peut citer notamment le gluconate de manganèse et autres sels, l'alvérine citrate et ses sels, la glycine, un extrait d'Iris pallida, un hexapeptide (Argériline R de Lipotec) ou les sapogénines comme le Wild yam et les amines carbonylées décrites dans la demande EP1484052.

Comme agents amincissants (lipolytiques), on peut citer notamment la théophylline et ses dérivés, la théobromine, l'acéfylline, l'aminophylline, le chloroéthylthéophylline, le diprofylline, le diniprophylline, l'étamiphylline et ses dérivés, l'étofylline, la proxyphylline ; les extraits de thé, de café, de guarana, de maté, de cola *(Cola Nitida)* et notamment l'extrait sec de fruit de guarana (*Paulina sorbilis*) contenant 8 à 10 % de caféine ; les extraits de lierre grimpant *(Hedera Helix),* d'arnica *(Arnica Montana L),* de romarin *(Rosmarinus officinalis N*), de souci *(Calendula officinalis),* de sauge *(Salvia officinalis L),* de ginseng *(Panax ginseng),* de millepertuis *(Byperycum Perforatum),* de fragon *(Ruscus aculeatus L),* d'ulmaire *(Filipendula ulmaria L),* d'orthosiphon *(Orthosiphon Stamincus Benth),* de bouleau *(Betula alba),* de cécropia et d'arganier ; les extraits de ginkgo biloba, les extraits de prêle, les extraits d'escine, les extraits de cangzhu, les extraits de *chrysanthellum indicum,* les extraits de dioscorés riches en diosgénine ou la diosgénine ou hécogénine pure et leurs dérivés, les extraits de Ballote, les extraits de *Guioa,* de *Davallia,* de *Terminalia,* de *Barringtonia,* de *Trema*, d'*Antirobia.*

Le ou les actifs et/ou ingrédients additionnels utilisés dans la composition B selon l'invention peuvent représenter de 0,0001 à 20%, de préférence de 0,01 à 10% et mieux, de 0,01 à 1 % en poids para rapport au poids total de la composition.

L'homme du métier saura choisir dans les listes précédentes les agents additionnels qui conviennent particulièrement au milieu basique de la composition B selon l'inventio et n'affectent pas les propriétés de l'hydroxyalkylurée.

Les compositions selon l'invention sont généralement adaptées à une application topique sur la peau et comprennent généralement un milieu physiologiquement acceptable, c'est-à-dire compatible avec la peau. Il s'agit d'un milieu cosmétiquement acceptable, c'est-à-dire qui présente une couleur, une odeur et un toucher agréables et qui ne génère pas d'inconforts inacceptables (picotements, tiraillements, rougeurs), susceptibles de détourner la consommatrice d'utiliser cette composition

Les compositions de l'invention peuvent se présenter sous toutes les formes appropriées pour une application topique, notamment sous forme de gels aqueux, sous forme d'émulsions obtenues par dispersion d'une phase grasse (appelée aussi phase huileuse) dans une phase aqueuse (H/E) ou inversement (E/H) ou d'émulsions multiples (par exemple E/H/E ou H/E/H ou H/H/E). Elles peuvent être plus ou moins fluides et avoir l'aspect d'une crème blanche ou colorée, d'une pommade, d'un lait, d'une lotion, d'un sérum, d'une pâte, de poudre, de bâtonnet solide et éventuellement être conditionnée en aérosol et se présenter sous forme de mousse ou de spray. Ces compositions sont préparées selon les méthodes usuelles.

Selon un mode particulier de réalisation de l'invention, les compositions de l'invention peuvent se présenter sous forme d'une émulsion et comporter alors au moins une phase huileuse. La proportion de la phase huileuse de l'émulsion peut aller de 1 à 80 % en poids, de préférence de 2 à 50 % en poids et mieux de 2 à 40 % en poids par rapport au poids total de la composition. Les corps gras de la phase huileuse, notamment les huiles, et les émulsionnants et co-émulsionnants éventuellement présents, utilisés dans la composition sous forme d'émulsion sont choisis parmi ceux classiquement utilisés dans le domaine cosmétique ou dermatologique. L'émulsionnant et le co-émulsionnant ,quand ils sont présents, le sont généralement, en une proportion allant de 0,1 à 30 % en poids, de préférence de 0,3 à 20 % en poids et mieux de 0,5 à 15 % en poids par rapport au poids de la composition. L'émulsion peut en outre, contenir des vésicules lipidiques en plus ou à la place des émulsionnants et/ou co-émulsionnants.

Les émulsions contiennent généralement au moins un émulsionnant choisi parmi les émulsionnants amphotères, anioniques, cationiques ou non ioniques, utilisés seuls ou en mélange. Les émulsionnants sont choisis de manière appropriée suivant la phase continue de l'émulsion à obtenir (E/H ou H/E). Quand l'émulsion est multiple, elle comporte généralement un émulsionnant dans l'émulsion primaire et un émulsionnant dans la phase externe dans laquelle est introduite l'émulsion primaire.

Comme émulsionnants utilisables pour la préparation des émulsions E/H, on peut citer par exemple les alkyl esters ou éthers de sorbitan, de glycérol ou de sucres ; les tensioactifs siliconés comme les dimethicone copolyols tels que le mélange de cyclomethicone et de dimethicone copolyol, vendu sous les dénominations DC 5225 C et DC 3225 C par la société Dow Corning, et comme les alkyl-dimethicone copolyols tels que le Laurylmethicone copolyol vendu sous la dénomination "Dow Corning 5200 Formulation Aid" par la société Dow Corning, le Cetyl dimethicone copolyol vendu sous la dénomination Abil EM 90^{R} par la société Goldschmidt et le mélange de Polyglyceryl-4 isostearate/Cetyl dimethicone copolyol/Hexyl laurate vendu sous la dénomination Abil WE 09^{R} par la société Goldschmidt. On peut y ajouter aussi un ou plusieurs co-émulsionnants, qui, de manière avantageuse, peuvent être choisis dans le groupe comprenant les esters d'acide gras à chaîne ramifiée et de polyol, et notamment les esters d'acide gras à chaîne ramifiée et de glycérol et/ou de sorbitan et par exemple l'isostéarate de polyglycéryle, tel que le produit commercialisé sous la dénomination Isolan GI 34 par la société Goldschmidt, l'isostéarate de sorbitan, tel que le produit commercialisé sous la dénomination Arlacel 987 par la société ICI, l'isostéarate de sorbitan et de glycérol, tel que le produit commercialisé sous la dénomination Arlacel 986 par la société ICI, et leurs mélanges.

Comme émulsionnants utilisables pour la préparation des émulsions H/E, on peut citer par exemple les émulsionnants non ioniques tels que les esters d'acides gras et de polyols oxyalkylénés (plus particulièrement polyoxyéthylénés), et par exemple les stéarates de polyéthylène glycol comme le stéarate de PEG-100, le stéarate de PEG-50 et le stéarate de PEG-40 ; les esters d'acides gras et de sorbitan oxyalkylénés comprenant par exemple de 20 à 100 OE, et par exemple ceux commercialisés sous les dénominations commerciales Tween 20 ou Tween 60 par la société Uniqema ; les éthers d'alcools gras oxyalkylénés (oxyéthylénés et/ou oxypropylénés) ; les esters de sucres, alcoxylés ou non, comme le stéarate de sucrose et comme le PEG-20 méthylglucose sesquistéarate ; les esters de sorbitan tels que le palmitate de sorbitan commercialisé sous la dénomination Span 40 par la société Uniqema ; les esters de diacide et d'alcool gras, tels que le tartrate de dimyristyle ; les mélanges de ces émulsionnants comme par exemple le mélange de stéarate de glycéryle et de stéarate de PEG-100 (nom CTFA : Glyceryl Stearate / PEG-100 Stearate) commercialisé sous la dénomination Arlacel 165 par la société Uniqema et sous la dénomination SIMULSOL 165 par la société SEPPIC ; ou le mélange de tartrate de dimyristyle, d'alcool cétéarylique, de Pareth-7 et de PEG-25 laureth-25, commercialisé sous la dénomination Cosmacol PSE par la société Sasol (nom CTFA : Dimyristyl tartrate / cetearyl alcool /12-15 Pareth 7 / PPG 25 laureth 25).

On peut ajouter à ces émulsionnants, des co-émulsionnants tels que par exemple les alcools gras ayant de 8 à 26 atomes de carbone, comme l'alcool cétylique, l'alcool stéarylique et leur mélange (alcool cétéarylique), l'octyl dodécanol, le 2-butyloctanol, le 2-hexyldécanol, le 2-undécylpentadécanol ou l'alcool oléique, ou les acides gras.

On peut aussi préparer des émulsions sans tensioactifs émulsionnants ou en contenant moins de 0,5 % du poids total de la composition (A) ou (B), en utilisant des composés appropriés, pour stabiliser lesdites émulsions par exemple des polymères amphiphiles, des électrolytes.

Quand les composition de l'invention sont sous forme d'émulsion, elles comportent au moins une phase huileuse qui contient au moins une huile, notamment une huile cosmétique. On entend par "huile" un corps gras liquide à la température ambiante (25°C).

Comme huiles utilisables dans la composition de l'invention, on peut utiliser par exemple les huiles hydrocarbonées d'origine animale, telles que le perhydrosqualène (ou squalane) ; les huiles hydrocarbonées d'origine végétale, telles que les triglycérides des acides caprylique/caprique comme ceux vendus par la société Stearineries Dubois ou ceux vendus sous les dénominations Miglyol 810, 812 et 818 par la société Dynamit Nobel ou encore les huiles d'origine végétale, par exemple les huiles de tournesol, de maïs, de soja, de courge, de pépins de raisin, de sésame, de noisette, d'abricot, de macadamia, d'arara, de coriandre, de ricin, d'avocat, l'huile de jojoba, l'huile de beurre de karité ; les huiles de synthèse ; les huiles de silicone comme les polyméthylsiloxanes (PDMS) volatiles ou non à chaîne siliconée linéaire ou cyclique, liquides ou pâteux à température ambiante ; les huiles fluorées telles que celles partiellement hydrocarbonées et/ou siliconées comme celles décrites dans le document JP-A-2-295912 ; les éthers tels que l'éther dicaprylique (nom CTFA : Dicaprylyl ether) ; et les benzoates d'alcools gras en C₁₂-C₁₅ (Finsolv TN de FINETEX) ; les dérivés arylalkyl benzoates comme le 2-phenylethyl benzoate (X-Tend 226 de ISP) ; les huiles amidées comme le N-lauroylsarcosinate d'isopropyle (ELDEW SL-205 de Ajimoto) et leurs mélanges.

Les compositions de l'invention peuvent également contenir un ou plusieurs solvants organiques qui peuvent être choisis dans le groupe constitué par les solvants organiques hydrophiles, les solvants organiques lipophiles, les solvants amphiphiles ou leurs mélanges.

Parmi les solvants organiques hydrophiles, on peut citer par exemple des alcools monohydriques linéaires ou ramifiés ayant de 1 à 8 atomes de carbone comme l'éthanol, le propanol, le butanol, l'isopropanol, l'isobutanol ; des polyéthylène glycols ayant de 6 à 80 oxydes d'éthylène ; des polyols tels que le propylène glycol, l'isoprène glycol, le butylène glycol, le glycérol, le sorbitol ; les mono- ou di-alkyle d'isosorbide dont les groupements alkyle ont de 1 à 5 atomes de carbone comme le diméthyl isosorbide ; les éthers de glycol comme le diéthylène glycol mono-méthyl ou mono-éthyl éther et les éthers de propylène glycol comme le dipropylène glycol méthyl éther.

Comme solvants organiques amphiphiles, on peut citer les dérivés de polypropylène glycol (PPG) tels que les esters de polypropylène glycol et d'acide gras, de PPG et d'alcool gras comme le PPG-23 oléyl éther et le PPG-36 oléate.

Comme solvants organiques lipophiles, on peut citer par exemple les esters gras tels que l'adipate de diisopropyle, l'adipate de dioctyle, les benzoates d'alkyle.

Les compositions conformes à la présente invention peuvent également comprendre des adjuvants cosmétiques classiques choisis parmi les adoucissants, les humectants, les opacifiants, les stabilisants, les émollients, les silicones, les agents anti-mousse, les parfums, les conservateurs, les tensioactifs anioniques, cationiques, non-ioniques, zwitterioniques ou amphotères, les charges, les polymères, les propulseurs, les agents alcalinisants ou acidifiants ou tout autre ingrédient habituellement utilisé dans le domaine cosmétique et/ou dermatologique.

Comme épaississants hydrophiles, on peut citer les polymères carboxyvinyliques tels que les carbopols (carbomers) et les Pemulen (Copolymère acrylate/C10-C30-alkylacrylate) ; les dérivés cellulosiques tels que l'hydroxyéthylcellulose; les polysaccharides et notamment les gommes telles que la gomme de xanthane ; et leurs mélanges.

Comme épaississants lipophiles, on peut citer les argiles modifiées telles que le l'hectorite et ses dérivés, comme les produits commercialisés sous les noms de Bentone.

L'homme du métier saura adapter le choix des adjuvants cosmétique et leurs teneurs en fonction du pH (acide ou basique) des compositions selon l'invention.

### Ensemble cosmétique

Les compositions A et B utilisées pour la mise en oeuvre de l'invention sont conditionnées séparément à l'intérieur de deux compartiments, formés soit par deux récipients distincts, soit à l'intérieur d'un dispositif unitaire.

Par « dispositif unitaire » on entend un dispositif par lequel les deux compartiments sont solidaires l'un de l'autre. Un tel dispositif peut être obtenu par un procédé de moulage en une seule pièce des deux compartiments, notamment en un matériau thermoplastique. Il peut également résulter de toute forme d'assemblage, notamment par collage, soudage, ou autre encliquetage.

Selon un premier mode de réalisation, les deux récipients sont indépendants l'un de l'autre. De tels récipients peuvent se présenter sous diverses formes. Il peut s'agir notamment de tubes, de flacons ou de bidons.

L'un et/ou l'autre des récipients peuvent être surmontés d'une pompe à actionnement manuel surmontée d'un bouton poussoir pour l'actionnement de la pompe et la distribution de la composition via au moins un orifice de distribution.

Alternativement, l'un et/ou l'autre des récipients sont pressurisés, notamment au moyen d'un agent propulseur, en particulier un gaz propulseur. Dans ce cas, le (ou les) récipient(s) est (sont) équipé(s) d'une valve surmontée par un bouton poussoir équipée d'une buse ou de tout autre moyen de diffusion pour la distribution du produit.

Le propulseur peut être en mélange avec la composition à distribuer ou séparé, notamment *via* un piston apte à coulisser à l'intérieur du récipient, ou *via* les parois souples d'une poche à l'intérieur de laquelle est disposée la composition.

Les récipients peuvent être constitués de matériaux divers : plastique, verre, ou métal.

Selon un mode de réalisation préféré, les deux compositions sont contenues à l'intérieur d'un dispositif unitaire.

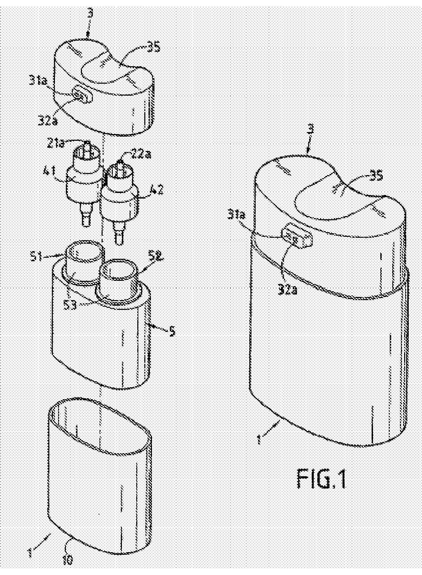

Selon le mode de réalisation représenté à la figure 1 , le dispositif de conditionnement 1 est constitué de deux compartiments 51, 52 disposés côte à côte et formés à l'intérieur d'une pièce 5 obtenue de moulage d'un matériau thermoplastique. Chacun des récipients 51, 52 comporte un col 53 délimitant une ouverture. A l'intérieur du col de chacun des récipients est montée une pompe 41, 42, qui peut être avec ou sans reprise d'air.

Lors du montage, la pièce 5 délimitant les deux compartiments 51, 52, est disposée à l'intérieur d'un élément d'habillage 10.

Une tige de pompe 21 a, 22a, de chacue des pompes 41, 42 est insérée à force à l'intérieur d'un conduit correspondant prévu dans un bouton poussoir unique 3 configuré de manière à permettre l'actionnement simultané des deux pompes, en réponse à une pression exercée axialement sur une surface 35 du bouton poussoir 3.

Les conduits du bouton poussoir connectés à chacune des pompes débouchent sur deux orifices 31a, 32a, disposés au voisinage l'un de l'autre sur une surface extérieure du bouton poussoir 3. En réponse à un actionnement des pompes 41, 42, les deux compositions sortent séparément soit sur le doigt de l'utilisatrice, soit sur un tampon ou coton applicateur. Le mélange des deux compositions se fait alors lors de l'application sur la surface à traiter.

Selon un autre mode de réalisation non illustré, les deux récipients sont pressurisés et équipés d'une valve à enfoncement ou basculement. Les deux valves sont de préférence actionnables par un même bouton poussoir du type de celui décrit en référence au mode de réalisation à pompes.

Alternativement encore, les deux compartiments sont formés de deux compartiments concentriques formés à l'intérieur d'un tube, et sont surmontés d'une pompe sans reprise d'air équipée d'un bouton poussoir à un ou deux orifices de distribution. A l'intérieur du tube est prévu un piston qui remonte en direction de la pompe au fur et à mesure que les compositions sont prélevées à l'intérieur des récipients. De tels modes de distribution sont utilisés notamment pour la distribution de pâtes dentifrices.

D'autres dispositifs encore peuvent être utilisés pour la mise en oeuvre de la présente invention, l'essentiel étant qu'ils puissent permettre le conditionemment séparé des deux compositions et leur distribution de manière séparée ou en mélange.

A titre d'exemple encore les deux compositions sont conditionnées à l'intérieur de deux compartiments formés à l'intérieur d'un même sachet souple, les deux compartiments étant séparés par une zone de rupture qui peut être cassée au moment de l'utilisation, notamment en réponse à une pression exercée à un endroit précis du sachet.

L'invention concerne donc en particulier un ensemble, dans lequel les compositions A et B sont conditionnées à l'intérieur de deux compartiments formés par deux récipients distincts.

Selon un mode particulier, les compositions A et B sont condititionnées à l'intérieur de deux compartiments (51, 52) délimités par un dispositif unitaire (1) tel que représenté à la figure 1.

En particulier, chacun des compartiments est équipé d'une pompe (41, 42), de préférence à actionnement manuel, reliée à au moins un moyen d'actionnement et de distribution (3) permettant de délivrer, séparément ou en mélange les compositions A et B.

Selon un mode préféré, le moyen d'actionnement et de distribution (3) est commun aux deux pompes.

Selon une alternative, chacun des compartiments est pressurisé, notamment au moyen d'un agent propulseur, et équipé d'une valve reliée à au moins un moyen d'actionnement et de distribution permettant de délivrer, séparément ou en mélange les compositions A et B.

En particulier, le moyen d'actionnement et de distribution est commun aux deux valves.

L'invention concerne également un procédé de soin de la peau et/ou du cuir chevelu comprenant l'application sur la peau ou le cuir chevelu d'une composition A et d'une composition B telle que définies précédemment, soit simultanément, soit de façon successive ou décalée dans le temps.

De préférence, l'application se fera de façon simultanée.

Selon un mode particulier, le procédé selon l'invention est destiné au soin des peaux grasses.

Selon un autre mode, le procédé selon l'invention est destiné au soin des peaux sèches, des peaux sensibles, des peaux délicates (ex : peaux de bébé) et/ou des peaux agressées (ex : rasage, traitement acnéique...).

L'invention va maintenant être décrite en référence aux exemples suivants donnés à titre illustratif et non limitatif. Dans ces exemples, sauf indication contraire, les quantités sont exprimées en pourcentages pondéraux.

### Exemple : Soin double effet astringent et hydratant

### Composition astringente A à pH 3,5 (acide) contenant des cristaux d'alun

| | |
|---|---|
| Alun de potassium | 1,5% |
| Ammonium polyacryloydimethyl taurate | |
| (Hostacerin AMPS^{®} de CLARIANT) | 2,0% |
| Silicones volatiles | 6,0% |
| Solvants | 4,0% |
| Acide stéarique | 1,0% |
| Emulsionnants | 3,0% |
| Conservateurs | 0,4% |
| Eau | qsp 100% |

### Composition hydratante B à pH 7 (basique) contenant de l'hydroxyalkylurée

| | |
|---|---|
| N-(2-hydroxyéthyl)-urée | 5,0% |
| Ammonium polyacryloydimethyl taurate | |
| (Hostacerin AMPS^{®} de CLARIANT) | 0,5% |
| Silicone volatile | 5,0% |
| Silicone polymérique (NLK506^{®} de Takemoto) | 3,0% |
| Glycérine | 3,0% |
| Emulsionnants | 1,5% |
| Conservateurs | 0,2% |
| Eau | qsp 100% |

Les compositions A et B sont préparées selon les techniques de formulation connues de l'homme du métier, puis sont conditionnées séparément dans un dispositif tel que décrit précédemment et représenté à la figure 1. Une pression exercée axialement sur bouton poussoir unique 3, actionne simultanément les deux pompes, et permet aux deux compositions A et B de sortir séparément sur le doigt de l'utilisatrice, ou sur un tampon ou coton applicateur. Le mélange des deux compositions se fait lors de l'application sur la surface à traiter.
On obtient après application sur la peau un bon effet astringent et hydratant.

### Test montrant l'instabilité du pH en présence de N-(2-hydroxyéthyl)-urée :

### 1) Composition de pH 6,4, contenant 5 % de N-(2-hydroxyéthyl)-urée

| | |
|---|---|
| N-(2-hydroxyéthyl)-urée | 5% |
| Caféine | 3% |
| Triethanolamine | 0,6% |
| Escine | 0,2% |
| Extrait de Ginkgo Biloba | 0,05% |
| Extrait de ruscus | 0,1% |
| Silice | 0,5% |
| Acide salicylique | 0,5% |
| Ammonium polyacryloydimethyl taurate | |
| (Hostacerin AMPS^{®} de CLARIANT) | 1,75% |
| Silicone volatile | 10% |
| Glycérine | 3% |
| Emulsionnants | 3% |
| Propylène glycol | 6% |
| Ethanol | 15% |
| Eau | qsp 100% |

Le pH est, au temps zéro, de 6,4, et il passe à 6,9 en 15 jours, ce qui montre l'instabilité du pH d'une composition de pH acide (pH inférieur à 7), contenant de la N-(2-hydroxyéthyl)-urée

### 2) Composition contenant une quantité croissante de N-(2-hydroxyéthyl)-urée

| | |
|---|---|
| N-(2-hydroxyéthyl)-urée | x% |
| Tetrasodium EDTA | 0,1% |
| Extrait de cacao | 1 % |
| Huiles | 18,5% |
| Sodium Acrylate/Sodium Acryloyldimethyl Taurate Copolymer / | |
| Isohexadecane / Polysorbate 80 (Simulgel EG de SEPPIC) | 1% |
| Sodium hyaluronate | 0,02% |
| Carbomer | 0,5% |
| Triéthanolamine | 0,25% |
| Divinyldimethicone/Dimethicone Copolymer /C12-13 Pareth-23 / | |
| C12-13 Pareth-3 (HMW2220 de DOW CORNING) | 0,5% |
| Glycérine | 10% |
| Emulsionnants | 2,5% |
| Ethanol | 3,5% |
| Conservateurs | 0,75% |
| Eau | qsp 100% |

| | Valeur de x% de N-(2-hydroxyéthyl)-urée | pH au temps zéro | pH après 15 jours à la température ambiante |
|---|---|---|---|
| 1 | 7 | 5 | 5,3 |
| 2 | 14 | 5 | 5,2 |
| 3 | 20 | 5 | 5,2 |
| 4 | 30 | 5 | 5,4 |
| 5 | 40 | 5 | 5,6 |

On constate que le pH après 15 jous a d'autatn plus varié que le pourcentage d'hydrovance est élevé.
Par ailleurs, après 15 jours à 45°C, la composition contenant 20 % d'hydrovance passe de pH 5,3 à pH 6,2. On observe donc une très forte instabilité du pH en présence d'hydrovance.

## Revendications

1. Ensemble pour le soin de la peau et/ou du cuir chevelu comprenant :
- une composition A contenant un milieu acide physiologiquement acceptable et au moins un agent de soin de la peau et/ou du cuir chevelu ;
- une composition B, conditionnée de manière séparée de la composition A, la composition B contenant un milieu basique physiologiquement acceptable et au moins une hydroxyalkylurée de formule (I) 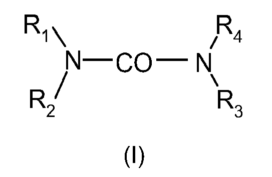
dans laquelle R₁, R₂, R₃ et R₄ représentent chacun indépendamment un atome d'hydrogène, un groupe alkyle en C₁-C₄ ou un groupe hydroxyalkyle en C₂-C₆ pouvant contenir de 1 à 5 groupes hydroxyle, au moins l'un des radicaux R₁-R₄ représentant un groupe hydroxyalkyle, ainsi que leurs sels, solvats et isomères.

2. Ensemble selon la revendication 1 **caractérisé en ce que** ledit agent de soin de la peau est autre qu'un autobronzant ou une enzyme.

3. Ensemble selon la revendication 1 ou 2 **caractérisé en ce que** l'agent de soin de la peau est présent dans la composition A en une teneur allant de 0,0001 à 90% en poids par rapport au poids total de la composition.

4. Ensemble selon l'une des revendications 1 à 3, **caractérisé en ce que** le pH du milieu acide est inférieur ou égal à 5.

5. Ensemble selon l'une quelconque des revendications 1 à 4, **caractérisé en ce que** R₁ est un groupe hydroxyalkyle et R₂, R₃ et R₄ représentent, indépendamment les uns des autres, un atome d'hydrogène ou un groupe alkyle en C₁-C₄.

6. Ensemble selon la revendication 5, **caractérisé en ce que** R₁ est un groupe hydroxyalkyle et R₂, R₃ et R₄ représentent chacun un atome d'hydrogène.

7. Ensemble selon l'une quelconque des revendications 1 à 6, **caractérisé en ce que** le composé de formule (I) est choisi parmi la N-(2-hydroxyéthyl)-urée ; la N-(2-hydroxypropyl)-urée ; la N-(3-hydroxypropyl)-urée ; la N-(2,3-dihydroxypropyl)-urée; la N-(2,3,4,5,6-pentahydroxyhexyl)-urée ; la N-méthyl-N-(1,3,4,5,6-pentahydroxy-2-hexyl)- urée ; la N-méthyl-N'-(1-hydroxy-2-méthyl-2-propyl)- urée ; la N-(1-hydroxy-2-méthyl-2-propyl)- urée ; la N-(1,3-dihydroxy-2-propyl)- urée ; la N-(tris-hydroxyméthyl-méthyl)-urée ; la N-éthyl-N'-(2-hydroxyéthyl)- urée ; la N,N-bis-(2-hydroxyéthyl)- urée ; la N,N'-bis-(2-hydroxyéthyl)- urée ; la N,N-bis-(2-hydroxypropyl)- urée ; la N,N'-Bis-(2-hydroxypropyl)- urée ; la N,N-Bis-(2-hydroxyéthyl)-N'-propyl- urée ; la N,N-Bis-(2-hydroxypropyl)-N'-(2-hydroxyéthyl)-urée ; la N-tert.Butyl-N'-(2-(hydroxyéthyl)-N'-(2-(hydroxypropyl)- urée ; la N-(1,3-dihydroxy-2-propyl)-N'-(2-hydroxyéthyl)- urée ; la N,N-Bis-(2-hydroxyéthyl)-N',N'-dimethyl- urée ; la N,N,N',N'-tetrakis-(2-hydroxyéthyl)- urée ; la N',N'-Bis-(2-hydroxyéthyl)-N',N'-bis-(2-hydroxypropyl)- urée.

8. Ensemble selon l'une quelconque des revendications 1 à 7, **caractérisé en ce que** l'hydroxyalkyl urée est la N-(2-hydroxyéthyl)-urée.

9. Ensemble selon l'une quelconque des revendications 1 à 8, **caractérisé en ce que** l'hydroxyalkylurée est présente dans la composition B en une teneur allant de 0,01 à 50% en poids par rapport au poids total de la composition et plus préférentiellement de 0,1 à 20 % en poids par rapport au poids total de la composition et encore plus préférentiellement de 0,1 à 10% en poids par rapport au poids total de la composition.

10. Ensemble selon l'une quelconque des revendications 1 à 9, **caractérisé en ce que** l'agent de soin présent dans la composition A est choisi parmi un acide organique, un agent dépigmentant, un agent desquamant, un agent anti-microbien, un agent apaisant et/ou anti-inflammatoire, un agent sébo-régulateur, un agent cicatrisant, un agent astringent, un agent amincissant et leurs mélanges.

11. Ensemble selon la revendication 10, **caractérisé en ce que** l'acide organique présent dans la composition A est choisi dans le groupe constitué par les α-hydroxy-acides, les β-hydroxy-acides, les α-céto-acides, les β-céto-acides, leurs dérivés et leurs mélanges.

12. Ensemble selon la revendication 10 ou 11, **caractérisé en ce que** l'acide organique présent dans la composition A est choisi dans le groupe constitué par l'acide ascorbique, l'acide kojique, l'acide caféique, l'acide salicylique et ses dérivés, l'acide citrique, l'acide lactique, l'acide méthyllactique, l'acide glucuronique, l'acide glycolique, l'acide pyruvique, l'acide 2-hydroxy-butanoïque, l'acide 2-hydroxypentanoïque, l'acide 2-hydroxyhexanoïque, l'acide 2-hydroxyheptanoïque, l'acide 2-hydroxyoctanoïque, l'acide 2-hydroxy-nonanoïque, l'acide 2-hydroxydécanoïque, l'acide 2-hydroxyundécanoïque, l'acide 2-hydroxydodécanoïque, l'acide 2-hydroxy-tétradécanoïque, l'acide 2-hydroxy-hexadécanoïque, l'acide 2-hydroxy-octadécanoïque, l'acide 2-hydroxytétra-cosanoïque, l'acide 2-hydroxy-eïcosanoïque, l'acide mandélique, l'acide benzoïque, l'acide phényllactique, l'acide gluconique, l'acide galacturonique, l'acide aleuritique, l'acide ribonique, l'acide tartronique, l'acide tartrique, l'acide malique, l'acide fumarique, l'acide rétinoïque et ses dérivés, l'acide benzène 1,4-di(3-méthylidène 10-camphosulfonique, l'acide urocanique, l'acide 2-phényl benzimidazole 5-sulphonique, l'acide α-(oxo-2-bornylidène-3) toluène-4-sulfonique, l'acide 2-hydroxy-4-méthoxy-5-sulfonique, les extraits végétaux contenant des acides et plus spécialement les extraits de fruits, les dérivés xanthiques acides, l'acide β-glycyrrhétinique, l'acide asiatique et leurs mélanges.

13. Ensemble selon la revendication 10, **caractérisé en ce que** l'agent de soin présent dans la composition A est choisi parmi l'acide ascorbique et ses dérivés, l'acide kojique, les sels de zinc, les sels de cuivre, l'acide n-octanoyl 5 salicylique, l'acide (N-2 hydroxyéthylpiperazine-N-2-éthane) sulfonique, les acides glycolique, citrique, lactique, tartrique, malique ou mandélique, les sels d'alun, un extrait d'Hammamelis, un extrait de rose, un extrait de menthe, l'acide β-glycyrrhétinique, l'allantoine, la sérine, l'arginine, l'hydroxyproline, la caféine, l'acide nicotinique et ses dérivés.

14. Ensemble selon l'une quelconque des revendications précédentes, **caractérisé en ce que** l'agent de soin est présent dans le milieu acide physiologiquement acceptable en une teneur allant de 0,001 à 90% en poids par rapport au poids total de la composition A, de préférence de 0,01 à 30% en poids par rapport au poids total de la composition A et encore plus préférentiellement de 0,05 à 10% en poids par rapport au poids total de la composition A.

15. Ensemble selon l'une quelconque des revendications précédentes, **caractérisé en ce que** la composition B comprend en outre au moins un agent additionnel de soin de la peau et/ou du cuir chevelu.

16. Ensemble selon l'une quelconque des revendications précédentes, **caractérisé en ce que** les compositions A et B sont conditionnées à l'intérieur de deux compartiments formés par deux récipients distincts.

17. Ensemble selon l'une quelconque des revendications 1 à 15 **caractérisé en ce que** les compositions A et B sont condititionnées à l'intérieur de deux compartiments (51, 52) délimités par un dispositif unitaire (1).

18. Ensemble selon la revendication 17 **caractérisé en ce que** chacun des compartiments est équipé d'une pompe (41, 42), de préférence à actionnement manuel, reliée à au moins un moyen d'actionnement et de distribution (3) permettant de délivrer, séparément ou en mélange les compositions A et B.

19. Ensemble selon la revendication 18 **caractérisé en ce que** le moyen d'actionnement et de distribution (3) est commun aux deux pompes.

20. Ensemble selon la revendication 17 **caractérisé en ce que** chacun des compartiments est pressurisé, notamment au moyen d'un agent propulseur, et équipé d'une valve reliée à au moins un moyen d'actionnement et de distribution permettant de délivrer, séparément ou en mélange les compositions A et B.

21. Ensemble selon la revendication 20 **caractérisé en ce que** le moyen d'actionnement et de distribution est commun aux deux valves.

22. Procédé de soin de la peau et/ou du cuir chevelu **caractérisé par le fait que** l'on applique sur la peau ou le cuir chevelu une composition A et une composition B telle que définies dans l'une quelconque des revendications 1 à 14 soit simultanément, soit de façon successive ou décalée dans le temps.

23. Procédé selon la revendication 22, **caractérisé en ce qu'**il est destiné au soin des peaux grasses.

24. Procédé selon la revendication 22, **caractérisé en ce qu'**il est destiné au soin des peaux sèches, des peaux sensibles, des peaux délicates et/ou des peaux agressées.
